Europäisches Patentamt

European Patent Office

Office européen des brevets

(19)

(11) Publication number: **0 081 727**
A2

# EUROPEAN PATENT APPLICATION

(12)

(21) Application number: 82110966.7

(22) Date of filing: 27.11.82

(51) Int. Cl.³: **A 61 L 11/00,** B 09 B 5/00,
B 01 F 15/00

(30) Priority: 15.12.81 IT 2561881

(43) Date of publication of application: 22.06.83
Bulletin 83/25

(84) Designated Contracting States: **AT BE CH DE FR GB LI
LU NL SE**

(71) Applicant: **INTEC S.R.L., Via S. Maria in Chiavica 4,
I-37121 Verona (IT)**

(72) Inventor: **Menegatto, Mario, Via XXV Aprile 12, Taglio di
Po (Rovigo) (IT)**

(74) Representative: **Gervasi, Gemma et al, Studio Brevetti e
Marchi NOTARBARTOLO & GERVASI 33, Viale Bianca
Maria, I-20122 Milano (IT)**

(54) Plant for treating solid urban refuse.

(57) A reactor is described for sterilising and drying urban
refuse at 120–130 °C.

The new reactor is essentially characterised by a cer-
tain number of retractable blades disposed on a central
rotating shaft and possibly also on its peripheral wall. The
purpose of these blades is to turn over the mass in order to
facilitate its sterilisation and drying. Discharge is facilitated
to a maximum extent by retracting them when the operation
is terminated.

- 1 -

PLANT FOR TREATING SOLID URBAN REFUSE

This invention relates to a new plant for treating urban refuse, characterised by comprising a reactor of new design for sterilising and drying the refuse.

The disposal of large accumulations of urban refuse is known to be an increasingly serious problem which has not yet been solved satisfactorily.

In particular, these accumulations represent a receptacle for germs and bacteria of all types, and thus constitute a serious potential danger for the initiation and propagation of infective processes. Consequently, the greatest problem posed by urban refuse, upstream of any recovery and/or utilisation process, is to sterilise and dry the accumulation so as to make any further treatment safe from a human and ecological viewpoint.

A plant for treating solid urban refuse has now been conceived, and forms the subject matter of the present invention, comprising a new type of reactor for sterilising and drying the refuse.

More specifically, a preferably vertical cylindrical reactor has been conceived for sterilising and drying the refuse, characterised by a central rotating shaft fitted with retractable blades, and by the presence of retractable blades in its side walls.

When extracted, the purpose of these blades is to turn over the mass so as to facilitate and accelerate its sterilisation and subsequent drying at all points. On termination of the operation, the blades are retracted so as not to obstruct the discharge of the mass which in general takes place downwards, and so as not to retain paper or fibrous refuse such as wire, string, rags etc.

In carrying out the sterilisation, the reactor is raised to a temperature of 120-130°C by steam circulating through a jacket or coil embracing the reactor. In addition, dry superheated steam is injected into the mass in order to accelerate the sterilisation operation. The sterilisation time varies with the reactor volume and thus with the volume of the mass treated. The drying stage, conducted at the same temperature, is preferably carried out under vacuum in order to reduce treatment time.

In this case, the treatment time also depends on the volume of mass treated, and is reduced by the pretreatment with dry superheated steam.

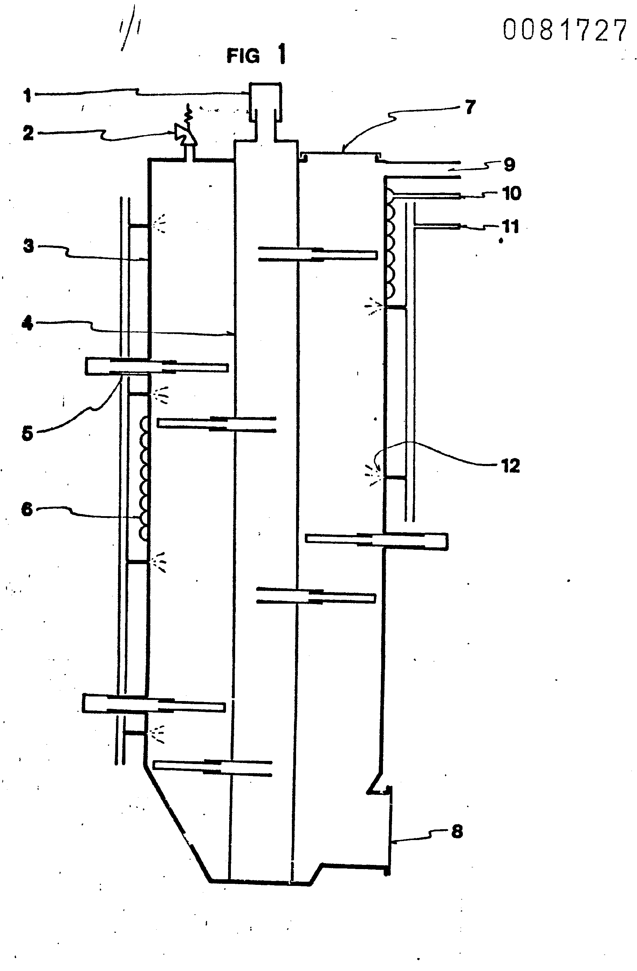

The new reactor according to the present invention will be more apparent from the description given hereinafter with reference to the accompanying drawing, in which the essential parts of the reactor are diagrammatically represented and identified by the following reference numerals:

3 represents the reactor

4 represents the central rotating shaft

7 represents the loading port

8 represents the discharge port

5 represents the retractable blades

6 represents the coil or jacket through which steam circulates

0081727

2 represents a safety valve

1 represents the connector which connects the retractable blade controls (not shown) to their hydraulic or pneumatic control system

9 represents the connector which connects the reactor to a system for creating a more or less high vacuum in its interior

10 represents the steam connection for the jacket or coil

11 represents the connection for the dry superheated steam injected into the interior of the mass

12 represents the dry superheated steam injectors or nozzles.

By way of example, the new reactor for sterilising and drying urban refuse, according to the invention, can have the following dimensions: diameter 1500 mm; height of cylindrical part 4000 mm; total reactor height 5500 mm; volume 8.5 $m^3$; useful capacity 7 $m^3$; shaft diameter 400 mm; shaft rotation motor 40 HP; loading port 700 mm; discharge port 500 mm. The retractable "blades" can be of any suitable shape, e.g. in the form of a rod of suitable diameter or a parallelepiped of suitable dimensions. In any case, the dimensions of the turning blades fixed to the shaft are influenced by the reactor diameter and diameter of the rotating shaft, in that they have to reach as close as possible to the reactor walls, but also have to be able to retract totally into the shaft.

The dimensions of the blades fixed along the reactor walls, their purpose being to exert a breaking-up action on the mass, are also influenced by the free annular space in the reactor. A corresponding space must obviously also be available on the outside of the reactor into which the blades can retract.

Preferably, but not necessarily, all the blades whether fixed to the shaft or to the reactor walls have the same shape and dimensions.

In the illustrative example, the blades have a length of 400 mm, the other dimensions being calculated such as to ensure sufficient strength, according to the material used.

The discharge port 8 is fitted with a suitable slide valve or equivalent discharge valve. Alternatively, the port can be situated at the bottom of the reactor under the shaft.

The following would represent an example of a typical operation carried out in the reactor: 6 $m^3$ of urban refuse of average specific gravity 0.5 are loaded through the port 7. Simultaneously, all the blades 5 are extracted and the shaft 4 is rotated at a speed of 15-30 r.p.m. Superheated steam at 120-130°C is fed into the coil or jacket 6 through a suitable steam connection 10. The steam is circulated and discharged in known manner.

The loading operation requires about 20 minutes.

The dry superheated steam is fed through the connection 11 and on to the mass by way of the nozzles 12.

The sterilisation operation can be considered complete in 30 minutes. The drying stage begins after this time, and is carried out preferably under vacuum applied through the connector 9, while superheated steam at 120-130°C continues to be circulated through the outer jacket. The drying stage can be considered complete after 60 minutes.

The shaft is then halted, the blades are retracted and the vacuum source disconnected. The discharge slide valve opens automatically, and the sterile dry mass passes rapidly into a prearranged storage vessel.

The storage vessel is preferably placed under vacuum in order to ensure rapid and complete discharge.

Two or more sterilisation and drying reactors disposed in series can be provided, according to the daily mass of refuse to be treated, and taking account of the aforesaid times. In this case, the superheated steam at 120-130°C leaving the first reactor at about 100°C is again superheated by a suitable heat exchanger to 150°C and fed to the second reactor.

Various modifications can be made to the new reactor according to the invention without leaving the scope of the inventive idea.

- 1 -

0081727

PATENT CLAIMS

1. A plant for treating urban refuse, characterised by comprising a cylindrical sterilisation and drying reactor comprising a central rotating shaft fitted with retractable blades.

2. A plant as claimed in claim 1, characterised in that the reactor also comprises retractable blades disposed on its peripheral surface.

3. A plant as claimed in claim 1, characterised in that the reactor is fitted with an outer jacket or coil through which steam at 120-130°C circulates.

4. A plant as claimed in claim 1, characterised in that the reactor comprises a connector through which vacuum can be applied to its interior.

5. A plant as claimed in claim 1, characterised in that the reactor comprises hydraulic or pneumatic controls which enable the blades to be extracted and retracted.

FIG 1

0081727